# EUROPÄISCHE PATENTSCHRIFT

(11) **EP 0 871 410 B1**
(45) Veröffentlichungstag und Bekanntmachung des Hinweises auf die Patenterteilung: **27.10.1999**
(21) Anmeldenummer: 96941037.2
(22) Anmeldetag: 27.11.1996
(51) Int. Cl.: A61C 13/23

(54) **HAFTBAND FÜR ZAHNPROTHESEN**
ADHESIVE STRIP FOR DENTURES
BANDE ADHESIVE POUR PROTHESES DENTAIRES

(30) Priorität: 02.12.1995 DE 19545027
(43) Veröffentlichungstag der Anmeldung: 21.10.1998
(73) Patentinhaber: Dietzel, Karl, Dr., 47829 Krefeld (DE)
(72) Erfinder: Dietzel, Karl, Dr., 47829 Krefeld (DE)
(74) Vertreter: Rehders, Jochen
(86) Internationale Anmeldenummer: EP9605232
(87) Internationale Veröffentlichungsnummer: WO9720520

(56) Entgegenhaltungen:
- EP-A- 0 353 375
- EP-A- 0 407 681
- WO-A-91/11153

## Beschreibung

Die Erfindung betrifft ein Haftband für Zahnprothesen, insbesondere für Unterkieferprothesen.

Gebißprothesen bestehen im allgemeinen aus einem die künstlichen Zähne haltenden, festen Kunststoffmaterial, das an das weiche Gewebe des Mundes und den Unterkieferbereich ohne natürliche Zähne in seiner Form angepaßt wird.

Wegen der sehr komplizierten Bewegung, vor allem des Unterkiefers, beim Kauen, ist es schwierig, eine gute Haftung der Prothese auf dem Unterkiefergewebe zu erreichen.

Gegenüber der Haftung einer Oberkieferprothese, für die als Anlagefläche der Gaumen zur Verfügung steht, ist die Haftung einer Unterkieferprothese erheblich schwieriger zu erreichen, da die Auflagefläche erheblich kleiner ist und wegen der komplizierten Kaubewegungen des Unterkiefers ganz andere physikalische Bedingungen für die Haftung vorliegen.

Zur Verbesserung der Haftung von Gebißprothesen werden Haftcremes oder Haftpuder verwendet, bei denen die Qualität der Haftung außer von der Zusammensetzung des Haftmittels auch von der auf die Prothese aufgebrachten Schichtdicke und von der Fläche des Haftmittels abhängt.

Hierbei ist auch zu beachten, daß sich aus der Physik des Klebens ergibt, daß die Haftung um so besser ist, je dünner eine Klebeschicht ist. Da das Haftmittel manuell auf die Unterkieferprothese aufgebracht wird, ist es sehr schwierig, die erforderliche Haftmittelmenge gleichmäßig und reproduzierbar auf die Gebißprothese aufzubringen, und es folgt daraus, daß ein in sehr dünner Schicht aufgebrachtes Haftmittel zwar eine sehr gute Soforthaftung bewirkt, die aber nur von geringer Dauer sein kann. Wird andererseits eine zu dicke Klebeschicht aufgetragen, wird das Haftmittel heim Kauvorgang seitlich herausgedrückt und behindert die auf die Gebißprothese angewiesene Person.

Des weiteren ist nachteilig, daß ein Teil des im allgemeinen wasserlöslichen Haftmittels vom Gebißträger regelmäßig im Verdauungstrakt aufgenommen wird, so daß gesundheitliche Spätfolgen bzw. Nebenwirkungen nicht auszuschließen sind.

Um die Haftung zu verbessern und eine möglichst geringe Menge des Haftmittels zu verwenden, sind bereits mit einem Haftmittel imprägnierte Hafteinlagen vorgeschlagen worden, deren Trägermaterial aus einem Gewebe oder einem Faservlies besteht. Diese Hafteinlagen werden entweder im voraus in der benötigten Form formgestanzt oder zugeschnitten, oder werden vom Gebißprothesenträger bei Bedarf zugeschnitten und in die benötigte Form gebogen. Eine derartige Hafteinlage ist in der GB-PS 460 887 beschrieben.

Das Zuschneiden und Anbringen dieser bekannten Hafteinlagen erfordert eine gewisse Geschicklichkeit, die häufig zu einer Erschwernis für den Gebißprothesenträger führt. Des weiteren ist die Produktion dieser Hafteinlagen mit erheblichen Abfall behaftet, da durch die Art der Schnittform Abfall bereits vorgegeben ist.

Ein weiterer erheblicher Nachteil dieser Art Hafteinlagen besteht darin, daß durch das Zuschneiden oder Ausstanzen von Vliesen oder Geweben Ränder mit freien Fadenenden und Fadenstücken entstehen, die sich von der Hafteinlage lösen und vom Gebißprothesenträger verschluckt werden, so daß sich gesundheitliche Probleme aufgrund ihrer chemischen und mechanischen Zusammensetzung ergeben können.

Schließlich hat sich auch herausgestellt, daß sich diese Hafteinlagen mit einem als Vlies oder Gewebe ausgebildeten Trägermaterial nur schwierig in die gewünschte dreidimensionale Form bringen lassen, so daß der Gebrauchswert letztendlich begrenzt ist.

Nachteilig ist des weiteren, daß das Haftmittel das Trägermaterial auf seiner gesamten Breite bis zum Rand gleichmäßig durchdringt und dadurch beim Kauen leicht seitlich herausgequetscht wird.

Der Erfindung liegt das Problem zugrunde, eine einfach handhabbare Hafteinlage zu schaffen, die bei der Herstellung und beim Gebrauch keine oder nur geringfügige Abfälle verursacht, wenige sich lösende Fadenstücke oder Faserenden aufweist und bei der sich das Haftmittel überwiegend im mittleren Bereich befindet, ohne daß die Gefahr besteht, daß es seitlich herausgequetscht wird.

Ausgehend von dieser Problemstellung wird bei einem Haftband für Zahnprothesen, insbesondere für Unterkieferprothesen, das aus einem mit einer der Oberfläche des Hohlraums in der Basisfläche der Prothese entsprechenden Breite hergestellten, mit einem Haftmittel imprägnierten Textilband besteht, vorgeschlagen, daß erfindungsgemäß bei einem gewebten Band die Kettfäden an den Außenränder dicht aneinanderliegen und deren Abstand zu den mittigen Kettfäden hin zunimmt, wobei die Schußfäden so beabstandet und angeordnet sind, daß sich ein dreidimensional verformbares Band mit fest eingebundenen Außenkettfäden ergibt, oder daß bei einem aus einer Maschenware bestehenden oder geflochtenen Band die Maschen an den Außenrändern dicht aneinanderliegen und deren Abstand zu den mittigen Maschen hin zunimmt, so daß sich ein dreidimensional verformbares Band mit dicht liegenden äußeren und weiter liegenden inneren Maschen ergibt.

Da die Breite des Haftbandes der benötigten Breite entspricht, braucht dieses Haftband nur auf Länge geschnitten zu werden, da die Längskanten des Haftbandes eine Web- oder Wirkkante oder eine Flechtkante aufweisen, aus der sich keine Fadenstücke oder Faserenden lösen können. Das das Trägermaterial bildende Band läßt sich leicht dreidimensional Verformen, ohne Falten zu werfen und läßt sich daher problemlos dem dreidimensional geformten Hohlraum der Zahnprothese anpassen, wenn das Gewebe, die Maschenware oder Flechtware wie erfindungsgemäß vorgesehen, entsprechend elastisch verformbar ist.

Da sich das Haftband in einem automatisierten Fertigungsprozeß mit einer für eine ausreichende Haftung bestimmten Haftmittelmenge imprägnieren läßt, läßt sich eine stets gleichbleibende Haftwirkung erzielen und gleichzeitig eine erhebliche Verminderung der vom Zahnprothesenträger zu resorbierenden Haftmittelmenge erreichen.

Das Haftband läßt sich kontinuierlich in beliebiger Länge weben, als Maschenware oder Flechtware herstellen und automatisch mit der erforderlichen Haftmittelschicht imprägnieren.

Nach dem Imprägnieren mit dem Haftmittel werden Haftbandstücke mit einer für jeden Anwendungsfall ausreichenden Länge abgelängt und automatisch hygienisch verpackt, so daß bei der Herstellung kein Abfall entsteht. Wenn der Zahnprothesenträger ein Haftband benötigt, entnimmt er es der Verpackung, schneidet es auf die für seine Zahnprothese erforderliche Länge, legt es in den dreidimensional geformten Hohlraum der Prothese und kann danach sofort die Zahnprothese wieder tragen.

Die genau dosierte Haftmittelmenge durchdringt das Haftband im wesentlichen senkrecht zur Haftbandebene, da es durch engliegenden Fäden oder Maschen am Ausbreiten in der Haftbandebene zu den Rändern hin gehindert wird und in den Zwischenräumen zwischen den Fäden verbleibt. Daher kommt es beim Kauen auch nicht zu einem seitlichen Austreten des Haftmittels aus dem Haftband, so daß der Zahnprothesenträger durch austretendes Haftmittel nicht beim Essen behindert oder geschmacklich gestört wird.

Die erfindungsgemäßen Haftbänder bleiben dauerelastisch und passen sich durch einfachen Druck faltenfrei der mit Wasser abgespülten und daher benetzten Zahnprothese an.

Vorzugsweise besteht das Gewebe, die Maschenware oder Flechtware des Haftbandes aus hydrophilen Fasern, die mit großer Oberfläche gewebt, als Maschenware oder Flechtware hergestellt sein können, so daß etwa 80 bis 90 % des Haftmittels im Haftband bis zum Ende der Benutzungsdauer verbleibt und somit nicht vom Patienten resorbiert werden muß.

Das noch nicht mit Haftmittel imprägnierte Haftband kann eine Breite von 6 bis 14 mm, vorzugsweise von 8 bis 10 mm aufweisen, bei einer Dicke von etwa 0,2 mm.

Als Haftmittel läßt sich eine handelsübliche Haftcreme verwenden, wobei ein gewebtes oder als Maschenware oder als Flechtware hergestelltes Haftband kontinuierlich mit der Haftcreme imprägniert und anschließend in einer Wirbelkammer mit einem Haftpulver beschichtet werden kann, um eine bis zum Gebrauch nicht klebende Oberfläche zu erhalten. Hiermit ist der zusätzliche Vorteil verbunden, daß durch das Einpudern der Oberfläche die Haftwirkung verbessert wird. Ebenso läßt sich das Haftband beidseitig mit abziehbaren Folien versehen, die sich unmittelbar vor Gebrauch entfernen lassen. Bei einem Haftband mit den vorstehend angegeben Maßen, genügt eine Imprägnierung des Haftbandes mit einer Haftmittelmenge von etwa 0,05 bis 0,1 g/cm², um eine ausreichend lange Haftdauer zu erreichen.

Diese Haftdauer kann erfahrungsgemäß zwischen 5 und 24 Stunden betragen, je nachdem welche Speisen der Zahnprothesenträger zu sich nimmt, wobei eine sehr gute Verträglichkeit, und ohne daß Druckstellen entstehen, gegeben ist.

Sowohl die Maschenware als auch die Flechtware lassen sich als Band oder als Schlauch herstellen, wobei der Schlauch zur Bildung des Haftbandes flachgedrückt wird.

Vorzugsweise kann eine Maschenware verwendet werden, die sich beim Zuschneiden auf die gewünschte Länge nicht aufziehen läßt.

Erfindungswesentlich ist, daß das Haftband bereits bei der Herstellung die Breite aufweist, mit der es verwendet wird, es somit nur in der Länge zugeschnitten zu werden braucht, so daß nur sehr wenig Abfälle entstehen. Da das Haftband feste Webkanten bzw. Wirkkanten oder Flechtkanten aufweist, können nur wenige freie Fadenenden an den Längsenden des Haftbands beim Zuschneiden entstehen, die sich dort nicht ohne weiteres lösen können, zumal sie auch durch das Haftmittel im Fadenverbund gehalten werden. Die auf das Haftband aufgebrachte Haftmittelmenge läßt sich genau dosieren, so daß sich eine stets gleichbleibende und hervorragende Haftung ergibt und kein überschüssiges Haftmittel vom Zahnprothesenträger resorbiert werden muß.

Die Erfindung wird nachstehend anhand zweier in der Zeichnung dargestellter Ausführungsbeispiele des näheren erläutert. In der Zeichnung zeigen:
- Fig. 1: ein als Gewebe hergestelltes Haftband und
- Fig. 2: ein als Maschenware hergestelltes Haftband.

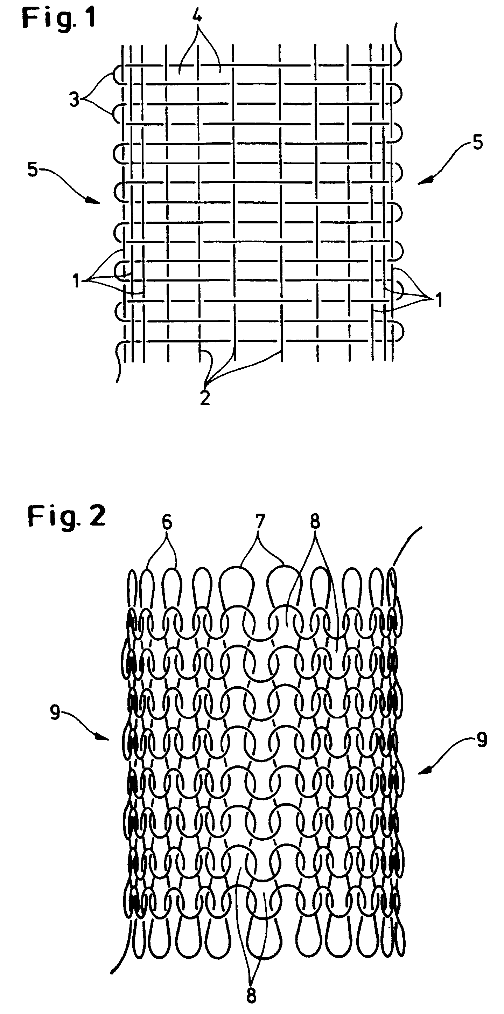

Das Haftband gemäß Fig. 1 ist ein Gewebeband, deren randnahe Außenkettfäden 1 dicht beieinander liegen, während die mittigen Kettfäden 2 einen größeren Abstand aufweisen. Schußfäden 3 sind mit einer solchen Bindung mit den Kettfäden 1, 2 verwebt, das sich ein dreidimensional verformbares Band mit an den Webkanten 5 fest eingebundenen Außenkettfäden 1 ergibt. Zwischen den Kettfäden 1, 2 und den Schußfäden befinden sich Zwischenräume 4, deren Größe von den äußeren Webkanten 5 zur Mitte hin zunimmt. Das nicht dargestellte Haftmittel füllt im wesentlichen die Zwischenräume 4 aus und wird durch die Fäden 1, 2, 3 in diesen Zwischenräumen 4 gehalten, so daß das Haftmittel zwar das Haftband im wesentlichen senkrecht zur Haftbandebene durchdringt, durch die Fäden 1, 2, 3 jedoch am Ausbreiten in der Haftbandebene gehindert wird und in den Zwischenräumen 4 verbleibt. Daher kommt es beim Kauen auch nicht zu einem seitlichen Austreten des Haftmittels aus dem Gewebeverbund des Haftbandes, so daß der Zahnprothesenträger durch austretendes Haftmittel nicht beim Essen behindert oder geschmacklich gestört wird.

In Fig. 2 ist ein als Maschenware hergestelltes Haftband dargestellt, bei der randnahe Maschen 6 enger gelegt sind als die Maschen 7 in der Mitte des Haftbandes. Auch hier sind die Fäden der Kanten 9 der Maschenware fest eingebunden, so daß keine Fasern oder Gewebeenden aus dem Haftband austreten können. Dieses als Maschenware hergestellte Haftband ist ebenfalls dreidimensional verformbar und weist Zwischenräume 8 zwischen den Fäden der Maschen 6, 7 auf, die zur Aufnahme des überwiegenden Teils des Haftmittels dienen. Vorzugsweise wird eine Maschenware verwendet, die sich nicht aufziehen läßt, wenn sie auf Länge geschnitten wird.

## Patentansprüche

1. Haftband für Zahnprothesen, insbesondere Unterkieferprothesen aus einem mit einer der Oberfläche des Hohlraums in der Basisfläche der Prothese entsprechenden Breite hergestellten, mit einem Haftmittel imprägnierten Gewebe, dadurch gekennzeichnet, daß es an den Außenrändern (5) dicht aneinanderliegende Kettfäden (1) aufweist, deren Abstand zu den mittigen Kettfäden (2) hin zunimmt, wobei die Schußfäden (3) so beabstandet und angeordnet sind, daß sich ein dreidimensional verformbares Band mit fest eingebundenen Außenkettfäden (1) ergibt, oder daß es aus einer Maschenware oder einer Flechtware mit an den Außenrändern (9) dicht aneinanderliegenden Maschen (6) besteht, deren Abstand zu den mittigen Maschen (7) hin zunimmt, so daß sich ein dreidimensional verformbares Band mit dicht liegenden äußeren und weiter entfernt liegenden inneren Maschen (6, 7) ergibt.

2. Haftband nach Anspruch 1, **dadurch gekennzeichnet**, daß das Gewebe oder die Maschenware oder die Flechtware aus hydrophilen Fasern besteht.

3. Haftband nach Anspruch 1 oder 2, **dadurch gekennzeichnet**, daß das Band eine Breite von 6 bis 14 mm, vorzugsweise von 8 bis 10 mm aufweist.

4. Haftband nach einem oder mehreren der Ansprüche 1 bis 3, **dadurch gekennzeichnet**, daß das Band eine Dicke von etwa 0,1 bis 0,3 mm hat.

5. Haftband nach einem oder mehreren der Ansprüche 1 bis 4, **dadurch gekennzeichnet**, daß das mit dem Haftmittel imprägnierte Band beidseitig mit einem Haftpulver beschichtet ist.

6. Haftband nach einem oder mehreren der Ansprüche 1 bis 5, **dadurch gekennzeichnet**, daß es beidseitig mit einer abziehbaren Folie versehen ist.

7. Haftband nach einem oder mehreren der Ansprüche 1 bis 6, **dadurch gekennzeichnet**, daß das Band mit einer Haftmittelmenge von etwa 0,05 bis 0,1 g/cm² imprägniert ist.

## Claims

1. Adhesive strip for dentures, in particular lower jaw dentures, made from a woven fabric impregnated with an adhesive and corresponding in width to the surface of the cavity in the base surface of the denture, **characterized in that** it features warp threads (1), arranged close together at the outer edges but becoming progressively further apart towards the central warp threads (2), the weft threads (3) being spaced and arranged in such a way that a three-dimensionally deformable strip with firmly tied outer threads (1) is produced, or in that it consists of a knitted or braided fabric with stitches arranged close together at the outer edges but becoming progressively further apart towards the central stitches (7) so that a three-dimensionally deformable strip with outer stitches arranged close together and more widely spaced inner stitches (6, 7) is produced.

2. Adhesive strip as claimed in Claim 1, **characterized in that** the woven or knitted or braided fabric consists of hydrophilic fibres.

3. Adhesive strip as claimed in Claim 1 or 2, **characterized in that** the strip has a width of 6 to 14 mm, preferably of 8 to 10 mm.

4. Adhesive strip as claimed in one or more of Claims 1 to 3, **characterized in that** the strip has a thickness of 0.1 to 0.3 mm.

5. Adhesive strip as claimed in one or more of Claims 1 to 4, **characterized in that** the strip impregnated with the adhesive is coated on both sides with an adhesive powder.

6. Adhesive strip as claimed in one or more of Claims 1 to 5, **characterized in that** it is equipped on both sides with a removable foil.

7. Adhesive strip as claimed in one or more of Claims 1 to 6, **characterized in that** the strip is impregnated with a quantity of adhesive of approximately 0.05 to 0.1 g/cm².

## Revendications

1. Bande adhésive pour prothèses dentaires, notamment pour prothèses de la mâchoire inférieure, faite en un tissu imprégné d'un produit adhésif, fabriqué dans une largeur correspondant à la surface de la cavité dans la surface de base de la prothèse, caractérisée en ce qu'elle présente, sur les bords extérieurs (5), des fils de chaîne (1) qui s'appliquent les uns tout contre les autres et dont la distance augmente vers les fils de chaîne (2) centraux, les fils de trame (3) étant espacés et disposés de manière qu'il se forme une bande déformable en trois dimensions avec des fils de chaîne extérieurs (1) fermement liés, ou en ce qu'elle consiste en un tricot ou un produit tressé avec des mailles (6) qui s'appliquent sur les bords extérieurs (9) les unes tout contre les autres et dont la distance augmente vers les mailles centrales (7), de sorte qu'il en résulte une bande déformable en trois dimensions avec des mailles extérieures (6) serrées et des mailles intérieures (7) plus éloignées.

2. Bande adhésive selon la revendication 1, caractérisée en ce que le tissu ou le tricot ou le produit tressé est constitué de fibres hydrophiles.

3. Bande adhésive selon la revendication 1 ou 2, caractérisée en ce que la bande présente une largeur de 6 à 14 mm, de préférence de 8 à 10 mm.

4. Bande adhésive selon une ou plusieurs des revendications 1 à 3, caractérisée en ce que la bande présente une épaisseur d'environ 0,1 à 0,3 mm.

5. Bande adhésive selon une ou plusieurs des revendications 1 à 4, caractérisée en ce que la bande imprégnée du produit adhésif peut être couverte sur les deux face d'une couche de poudre adhésive.

6. Bande adhésive selon une ou plusieurs des revendications 1 à 5, caractérisée en ce qu'elle est pourvue sur les deux faces d'une feuille pouvant être retirée.

7. Bande adhésive selon une ou plusieurs des revendications 1 à 6, caractérisée en ce que la bande est imprégnée d'une quantité de produit adhésif d'environ 0,05 à 0,01 g/cm².
